# EUROPÄISCHE PATENTSCHRIFT

(11) **EP 1 063 521 B1**
(45) Veröffentlichungstag und Bekanntmachung des Hinweises auf die Patenterteilung: **18.04.2007**
(21) Anmeldenummer: 00112129.2
(22) Anmeldetag: 06.06.2000
(51) Int. Cl.: G01N 27/83

(54) **Vorrichtung zur Prüfung von Rohrleitungen aus ferromagnetischen Materialien**
Device for testing ferromagnetic tubes
Appareil pour tester des tubes ferromagnétiques

(30) Priorität: 25.06.1999 DE 19929072
(43) Veröffentlichungstag der Anmeldung: 27.12.2000
(73) Patentinhaber: PII Pipetronix GmbH, 76297 Stutensee (DE)
(72) Erfinder: Laursen, Poul, Don Mills, Ontario, Canada M3B 1C7 (CA); Meredith, Christopher, Georgetown, Ontario, Canada 17G 1Y6 (CA)
(74) Vertreter: Lempert, Jost

(56) Entgegenhaltungen:
- EP-A- 0 775 910
- US-A- 4 649 343
- US-A- 5 537 035

## Beschreibung

Die Erfindung betrifft eine Vorrichtung zur Prüfung von Rohrleitungen aus ferromagnetischen Materialien, wie Pipelines, auf Fehler, Risse, Korrosion od. dgl., wie ein Prüfmolch, mit wenigstens einem Zugteil, einer am Zugteil angeordneten, jeweils um senkrecht zur Längsmittelachse des Zugteils angeordnete Achsen schwenkbare, im wesentlichen strahlenartig angeordnete Tragarme aufweisenden Tragstruktur mit veränderbarem Umfang und mehreren, am Umfang der Tragstruktur angeordneten Permanentmagneten zur Erzeugung eines Magnetfeldes und Sensoren.

Zum Prüfen von Rohrleitungen, insbesondere für den Wasser-, Öl- oder Gastransport, sind sogenannte Prüfmolche bekannt, die Prüfeinrichtungen mit am Außenumfang angeordneten Prüfelementen oder Sensoren aufweisen, mittels derer der Zustand der Wandung der Rohrleitung überprüft werden kann. Die Sensoren können in verschiedenartiger Weise ausgestaltet sein; es sind unter anderem piezoelektrische, elektroakustische, elektromagnetische Sensoren, wie Hall-, Streufluß- und Wirbelstromsensoren, bekannt.

Unterschiedliche Wandungszustände bzw. Wandstärkenschwächungen, beispielsweise aufgrund von Korrosion etc., liefern unterschiedliche Signale, die z.B. in einer Elektronikeinheit weiterverarbeitet werden können.

Um derartige Prüfmolche für die Prüfung von Rohrleitungen mit verschiedenen Normweiten verwenden bzw. die Prüfmolche über Zuführleitungen in die zu prüfende Rohrleitung einschleusen zu können, sind Prüfmolche mit an einem zentralen Zugteil angeordneten, radial expandierbaren Tragstrukturen bekannt, an deren Umfang die Prüfelemente und/oder Sensoren angeordnet sind. Solche Tragstrukturen mit in Abhängigkeit vom Innenquerschnitt der Rohrleitung veränderbarem Umfang weisen beispielsweise mehrere, um senkrecht zur Längsmittelachse des Zugteils angeordnete Achsen schwenkbare, im wesentlichen strahlenartig angeordnete Tragarme auf (DE 197 46 510 A1, DE 197 46 511 A1).

Die EP 0 775 910 A1 beschreibt eine Vorrichtung zur Prüfung ferromagnetischer Materialien, insbesondere Rohrleitungen, mit einer in Verbindung mit einem Magnetfeld zur Anregung bzw. Detektion von Ultraschallwellen dienenden Hochfrequenzstromspule, wobei das Magnetfeld im wesentlichen von um den Umfang der Rohrleitung angeordneten Permanentmagneten erzeugt wird. Darüber hinaus ist eine zusätzliche, ein magnetisches Vorfeld erzeugende Magnetanordnung vorgesehen.

Bei der Verwendung derartiger, auf einem elektromagnetischen Meßprinzip beruhender Vorrichtungen der eingangs genannten Art ist insbesondere nachteilig, daß das Magnetfeld der am Umfang der Tragstruktur angeordneten Permanentmagnete von deren lateralen Abstand bzw. von dem Querschnitt der jeweiligen Rohrleitung abhängt, wobei die Stärke des Magnetfelds umso höher bzw. die magnetische Felddichte umso größer ist, je geringer der laterale Abstand der Permanentmagnete bzw. je geringer der Querschnitt der jeweiligen Rohrleitung ist. Folglich nimmt die Meßempfindlichkeit bei Zunahme des Querschnitts der jeweiligen Rohrleitung ab. Weiterhin werden für Rohrleitungen mit unterschiedlichem Querschnitt keine vergleichbaren Meßergebnisse erhalten und sind diese mit unterschiedlichen Meßungenauigkeiten behaftet.

Der Erfindung liegt die Aufgabe zugrunde, hier auf konstruktiv einfache und kostengünstige Weise Abhilfe zu schaffen.

Erfindungsgemäß wird diese Aufgabe mit einer Vorrichtung der eingangs genannten Art dadurch gelöst, daß zumindest einigen Permanentmagneten ein weiterer Magnet zugeordnet ist, dessen Magnetfeld veränderbar ist, um das von dem Permanentmagneten erzeugte Magnetfeld in Abhängigkeit vom Umfang der Tragstruktur bzw. in Abhängigkeit vom lateralen Abstand der Permanentmagnete zu verstärken und/oder abzuschwächen.

Bei einer erfindungsgemäß ausgebildeten Vorrichtung kann das von den Permanentmagneten erzeugte Magnetfeld dadurch verstärkt oder abgeschwächt werden, indem die Richtung und/oder die Stärke des Magnetfelds der den Permanentmagneten zugeordneten Magnete verändert wird. Derart kann ein vom lateralen Abstand der Permanentmagnete unabhängiges Magnetfeld mit im wesentlichen konstanter magnetischer Felddichte für einen beliebigen Querschnitt der jeweiligen Rohrleitung erreicht werden. Aus Symmetriegründen ist insbesondere jedem Permanentmagnet ein Magnet mit veränderbarem Magnetfeld zugeordnet.

Eine erste Ausführungsvariante sieht vor, daß der Magnet mit veränderbarem Magnetfeld ebenfalls als Permanentmagnet ausgebildet und mittels eines Stellglieds drehbar ist, um die Richtung seines Magnetfeldes zu verändern. Hierbei können die Pole der Magnete durch Drehen derselben beispielsweise in eine der Ausrichtung der Pole der Permanentmagnete entsprechende Ausrichtung gebracht werden, um das von den Permanentmagneten erzeugte Magnetfeld zur Prüfung von Rohrleitungen mit großer Normweite maximal zu verstärken. Umgekehrt kann das von den Permanentmagneten erzeugte Magnetfeld beispielsweise dadurch maximal abgeschwächt werden, daß die drehbaren Magnete mit ihren Polen bis in eine den Polen der Permanentmagnete entgegensetzte Ausrichtung gebracht werden, um das von dem Permanentmagneten erzeugte Magnetfeld zur Prüfung einer Rohrleitung mit geringer Normweite abzuschwächen. Durch Drehen der Magnete können die Feldlinien ihrer Magnetfelder unter einem beliebigen Winkel zu den Feldlinien der von den Permanentmagneten erzeugten Magnetfelder ausgerichtet und letztgenannte dadurch unterschiedlich verstärkt bzw. abgeschwächt werden.

Das Stellglied kann wenigstens ein mit dem drehbaren Magnet kämmendes Zahnrad aufweisen, beispielsweise als drehfest mit einem solchen Zahnrad verbundene, drehbar gelagerte Welle ausgebildet sein. Der drehbare Magnet weist in diesem Fall ebenfalls entweder ein drehfest mit ihm verbundenes Zahnrad auf, oder der beispielsweise zylindrisch ausgebildete, drehbare Magnet ist am Umfang seiner Mantelfläche mit einer Verzahnung versehen.

Eine bevorzugte Ausführung sieht vor, daß das Stellglied elektrisch angetrieben ist. Das Stellglied kann beispielsweise mittels eines Elektromotors angetrieben sein, der mit wenigstens einem Sensorelement zur Ermittlung des Umfangs der Tragstruktur bzw. des lateralen Abstands der Permanentmagnete in Verbindung steht.

Gemäß einer anderen bevorzugten Ausführungsform ist das Stellglied mechanisch angetrieben. Ein solcher, rein mechanischer Antrieb hat insbesondere den Vorteil, daß er keine zusätzlichen, insbesondere elektrischen Antriebsmittel erfordert. Auf diese Weise ist er einerseits sehr kostengünstig, andererseits macht er die Anordnung von zusätzlichen Antriebsmitteln bzw. zusätzlichen Stromversorgungsmitteln entbehrlich.

In bevorzugter Ausführung ist das mechanisch angetriebene Stellglied mittels wenigstens einer Schraubenfeder angetrieben, die am Umfang der Tragstruktur die Tragarme laterial elastisch nach außen vorbelastet und bei einem lateralen Annähern der Tragarme bzw. einem lateralen Entfernen der Tragarme die mit ihrer Kompression bzw. Expansion verbundene Längenänderung in eine Drehbewegung des Stellglieds umsetzt.

Gemäß einer zweiten Ausführungsvariante ist vorgesehen, daß der Magnet mit veränderbarem Magnetfeld als Elektromagnet, wie eine Induktionsspule, ausgebildet und mit einem variablen Strom beaufschlagbar ist, um die Stärke seines Magnetfeldes zu verändern. In diesem Fall können die als Induktionsspulen ausgebildeten Magnete beispielsweise mit einem solchen Induktionsstrom beaufschlagt werden, der ein in Richtung des Magnetfeldes der Permanentmagnete gerichtetes Magnetfeld induziert, um dieses zur Prüfung einer Rohrleitung mit großer Normweite zu verstärken. Umgekehrt kann das von den Permanentmagneten erzeugte Magnetfeld dadurch abgeschwächt werden, daß die Induktionsspule mit einem entgegengesetzten Induktionsstrom beaufschlagt wird, um das von den Permanentmagneten erzeugte Magnetfeld zur Prüfung einer Rohrleitung mit kleiner Normweite abzuschwächen. Die Induktionsspulen stehen insbesondere mit wenigstens einem Sensorelement zur Ermittlung des Umfangs der Tragstruktur bzw. des lateralen Abstands der Permanentmagnete in Verbindung, um Stärke und/oder Richtung des Induktionsstroms je nach Querschnitt der Rohrleitung zu verändern.

Den am Umfang der Tragstruktur angeordneten Permanentmagneten ist bevorzugt jeweils in Längsrichtung ein weiterer Permanentmagnet zur Erzeugung eines im wesentlichen parallel zur Längsmittelachse des Zugteils verlaufenden Magnetfeldes zugeordnet. Auf diese Weise wird um den gesamten Mantelumfang des zu prüfenden Rohrabschnittes ein im wesentlichen homogenes Magnetfeld mit im wesentlichen parallelen Feldlinien erzeugt. Zweckmäßig ist beiden Permanentmagneten jeweils ein weiterer Magnet mit in Richtung und/oder Stärke veränderbarem Magnetfeld zugeordnet.

Die jeweils von einem Permanentmagnet und einem diesen zugeordneten Magnet mit veränderbarem Magnetfeld gebildeten Magnetisierungseinheiten sind insbesondere an Parallelogrammträgern angeordnet, die jeweils an zwei hintereinander angeordneten Tragarmen angelenkt sind. Derart ist sichergestellt, daß die magnetischen Feldlinien stets in identischer Richtung bezüglich der zu prüfenden Wandung der Rohrleitung, beispielsweise im wesentlichen parallel zur Längsmittelachse der Rohrleitung verlaufen.

Die Parallelogrammträger sind vorzugsweise mittels Federwangen elastisch lateral nach außen vorbelastet, so daß sich die Tragstruktur automatisch an Rohrleitungen mit verschiedenen Querschnitten anpaßt.

In bevorzugter Ausführung ist vorgesehen, daß die Federwangen zur Aufnahme mehrerer lateral beabstandeter Sensoren zur Überprüfung des Zustandes der Wandung der Rohrleitung dienen. Die Sensoren sind insbesondere mittels Federelementen zwischen den Federwangen angeordnet, wobei der laterale Abstand der Sensoren bei jedem Umfang der Tragstruktur jeweils konstant ist. Somit ist eine größtmögliche Meßgenauigkeit und Meßempfindlichkeit der erfindungsgemäßen Vorrichtung sichergestellt, indem einerseits die Magnetisierungseinheiten für ein vom Durchmesser der Rohrleitung im wesentlichen unabhängiges Magnetfeld und die unter jeweils konstanter lateraler Beabstandung angeordneten Sensoren für die Detektion kleinster, durch unterschiedliche Wandungszustände der Rohrleitung verursachter Signale sorgen.

Alternativ oder zusätzlich können auch die Tragarme mit einer Federkraft, beispielsweise mittels jeweils eines Tellerfederpakets, radial nach außen vorgespannt sein.

Vorzugsweise weist jeder Tragarm an seinem äußeren Ende im Bereich der Schwenkachse des Parallelogrammträgers und/oder jeder Parallelogrammträger wenigstens eine Stützrolle zur Führung an der Innenwand einer Rohrleitung auf.

Eine Weiterbildung der erfindungsgemäßen Vorrichtung sieht vor, daß die Tragarme und/oder die Parallelogrammträger synchronisiert sind. Derart ist die Vorrichtung insbesondere auch für solche Rohrleitungen bzw. Pipelines geeignet, welche beliebige, z.B. Y- oder T-förmige Verzweigungen aufweisen, indem beim Durchlauf eines Prüfmolches durch solche Verzweigungen diejenigen radial nach außen vorgespannten Tragarme bzw. Parallelogrammträger, die aufgrund der Verzweigung keinen Gegendruck von der Rohrinnenwand erfahren, von den benachbarten Tragarmen bzw. Parallelogrammträgern, welche an der der Verzweigung gegenüberliegenden Rohrwand anliegen, gehalten sind, so daß die freien Tragarme bzw. Parallelogrammträger nicht radial ausfedern können und somit eine Beschädigung der insbesondere an den Parallelogrammträgern angeordneten Prüfelemente zuverlässig verhindert wird. Außerdem wird auf diese Weise beim Durchlauf des Prüfmolches durch Rohrverzweigungen das zur Prüfung erforderliche Magnetfeld nicht beeinträchtigt. Vorzugsweise sind jeweils zwei benachbarte Tragarme und/oder zwei benachbarte Parallelogrammträger unter Gewährleistung eines um zwischen 1° und 5°, insbesondere zwischen 2° und 3°, voneinander abweichenden Winkels der Tragarme zur Längsmittelachse des Zugteils synchronisiert.

In bevorzugter Ausführung sind die Tragarme der Tragstruktur über eine am Zugteil festlegbare Zentralhülse an diesen befestigt. Hierbei kann die Zentralhülse, beispielsweise über ein Kugelgelenk, radial schwenkbar am Zugteil festgelegt sein, um den Durchlauf des Prüfmolchs durch Rohrkrümmungen zu erleichtern.

Nachfolgend ist die Erfindung anhand bevorzugter Ausführungsformen unter Bezugnahme auf die Zeichnungen näher erläutert. Es zeigen:
- Fig. 1: eine Seitenansicht einer Ausführungsform einer erfindungsgemäßen Vorrichtung mit einer Parallelogrammträger aufweisenden Tragstruktur;
- Fig. 2: eine Seitenansicht einer Ausführungsform eines Parallelogrammträgers;
- Fig. 3: eine Draufsicht auf einen Parallelogrammträgers gemäß Fig. 2 bei kleinem Umfang der Tragstruktur;
- Fig. 4: eine Draufsicht auf einen Parallelogrammträgers gemäß Fig. 2 bei großem Umfang der Tragstruktur;
- Fig. 5: einen Detailschnitt durch einen Parallelogrammträger gemäß Fig. 2 in Seitenansicht;
- Fig. 6: eine Seitenansicht eines elektrisch angetriebenen Stellglieds zur Änderung der Richtung des Magnetfeldes eines Magneten mit veränderbarem Magnetfeld;
- Fig. 7: eine Draufsicht auf ein Stellglied gemäß Fig. 6;
- Fig. 8: eine perspektivische Ansicht eines mechanisch angetriebenen Stellglieds zur Änderung der Richtung des Magnetfeldes eines Magneten mit veränderbarem Magnetfeld;
- Fig. 9: eine Seitenansicht eines Stellglieds gemäß Fig. 8;
- Fig. 10: eine schematische Ansicht eines Stellglieds gemäß Fig. 8 und 9;
- Fig. 11: eine Draufsicht auf ein Stellglied gemäß Fig. 8 bis 10;
- Fig. 12: eine Seitenansicht einer Schraubenfeder für ein Stellglied gemäß Fig. 8 bis 11 und
- Fig. 13: eine Seitenansicht einer Schraubenfeder gemäß Fig. 12 in Richtung Pfeil A.

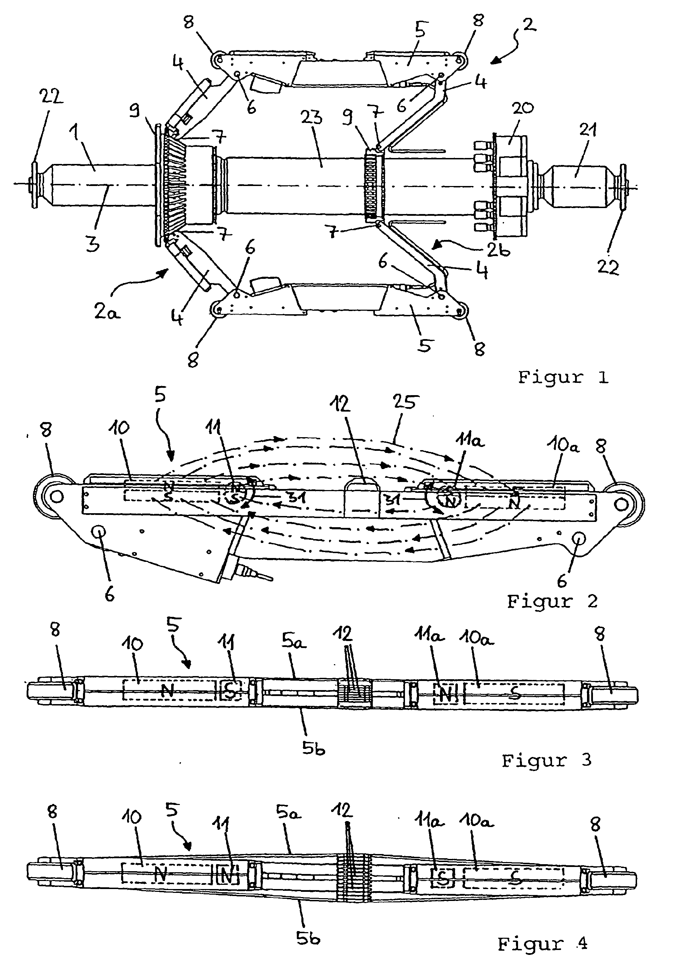

Die in Fig. 1 gezeigte Vorrichtung zur Prüfung von Rohrleitungen, wie Pipelines, auf Fehler, Risse, Korrosion od. dgl., weist ein Zugteil 1 und eine an diesem angeordnete Tragstruktur 2 auf. Die Tragstruktur 2 weist zwei hintereinander angeordnete Manschetten 2a, 2b mit jeweils im wesentlichen strahlenartig angeordneten, um senkrecht zur Längsmittelachse 3 des Zugteils 1 angeordnete Achsen 7 schwenkbaren Tragarmen 4 auf. Die Tragarme 4 jeder Manschette 2a, 2b sind jeweils über eine an einem Zentralkörper 23 angeordnete Zentralhülse 9 am Zugteil 1 festgelegt. Jede Manschette 2a, 2b kann z.B. mit zwölf Tragarmen 4 versehen sein. Jeder Tragarm 4 der Manschette 2a ist mit jeweils einem Tragarm 4 der Manschette 2b über einen Parallelogrammträger 5 verbunden, der an endseitig an den Tragarmen 4 angeordneten Schwenkachsen 6 parallel zur Längsmittelachse 3 des Zugteils 1 angelenkt ist. Die Tragarme 4 sind beispielsweise mittels jeweils eines (nicht dargestellten) Tellerfederpakets radial nach außen vorgespannt, so daß sich an den Parallelogrammträgern 5 angeordnete Stützrollen 8 stets an der Innenseite von (nicht dargestellten) Rohrleitungen mit unterschiedlichen Querschnitten abstützen. Die Zentralhülsen 9 können entweder fest mit dem Zugteil 1 verbunden sein, oder eine oder beide Zentralhülsen 9 sind, z.B. mittels eines Kugellagers, radial schwenkbar am Zugteil 1 festgelegt, um den Durchlauf der Vorrichtung durch Rohrkrümmungen zu erleichtern. Die Tragarme 4 und/oder die Parallelogrammträger 5 sind synchronisiert, um ein Ausfedern einzelner Tragarme 4 bzw. einzelner, an den Tragarmen 4 angelenkter Parallelogrammträger 5, z.B. bei dem Durchlauf der Vorrichtung durch Y- oder T-förmige Rohrverzweigungen, zu verhindern. Hierbei sind insbesondere jeweils zwei benachbarte Tragarme 4 und/oder Parallelogrammträger 5 unter Gewährleistung eines um zwischen 1° und 5° voneinander abweichenden Winkels der Tragarme 4 zur Längsmittelachse 3 des Zugteils 1 synchronisiert.

Um die Vorrichtung beispielsweise mit einem Molch zum Bewegen derselben durch eine Rohrleitung zu verbinden, sind an beiden Enden des Zugteils 1 Kupplungsstücke 22 angeordnet, von denen z.B. das rechte Kupplungsstück über eine schwenkbare Kupplungseinrichtung 21 und eine Verbindungseinrichtung 20 mit dem Zugteil 1 verbunden ist.

Wie aus Fig. 2 ersichtlich, sind an jedem Parallelogrammträger 5 zwei Permanentmagnete 10, 10a angeordnet, die ein mit den Feldlinien 25 angedeutetes Magnetfeld erzeugen, um Fehler, Risse oder Korrosion der Rohrleitung, welche das Magnetfeld beeinflussen, mittels ebenfalls an den Parallelogrammträgern 5 angeordneten elektromagnetischen Sensoren 12, wie Hall-, Streufluß-, Wirbelstromsensoren od. dgl., feststellen und die unterschiedlichen Signale gegebenenfalls in einer (nicht dargestellten) Elektronikeinheit weiterverarbeiten zu können. Die magnetischen Feldlinien 25 verlaufen im wesentlichen parallel zur Längsmittelachse des Zugteils. Um das von den Permanentmagneten 10, 10a erzeugte Magnetfeld in Abhängigkeit vom Umfang der Tragstruktur 2 bzw. in Abhängigkeit vom Innenquerschnitt der Rohrleitung zu verstärken und/oder abzuschwächen und somit für ein im wesentlichen vom lateralen Abstand der auf zwei benachbarten Parallelogrammträger 5 angeordneten Permanentmagnete 10, 10a unabhängiges Magnetfeld mit im wesentlichen konstanter Stärke zu sorgen, ist jedem Permanentmagnet 10, 10a ein weiterer Magnet 11, 11a zugeordnet, der in der gezeigten Ausführungsform ebenfalls als Permamentmagnet ausgebildet ist und dessen Magnetfeld durch Änderung seiner Richtung, nämlich durch Drehen des Magnets 11, 11a in Richtung Pfeil 31, veränderbar ist. In der in Fig. 2 dargestellten Position entspricht die Ausrichtung der Magnetpole (N, S) der Magnete 11, 11a der Ausrichtung der Magnetpole (N, S) der Permamentmagnete 10, 10a, wodurch deren Magnetfeld maximal verstärkt wird, wie es z.B. in Rohrleitungen mit einem großen Querschnitt bzw. bei großem lateralen Abstand der Permamentmagnete 10, 10a, erforderlich ist.

In Fig. 3 und 4 ist jeweils eine Draufsicht auf einen solchen Parallelogrammträger 5 dargestellt, der in der gezeigten Ausführungsform mittels seitlich angeordneten Federwangen 5a, 5b lateral nach außen vorbelastet ist, so daß die flexibel ausgebildeten Federwangen 5a, 5b sowohl in Rohrleitungen mit kleinem Querschnitt (Fig. 3), z.B. mit einem Querschnitt von 28', als auch in Rohrleitungen mit großem Querschnitt (Fig. 4), z.B. mit einem Querschnitt von 42', einander anliegen. Zwischen den Federwangen 5a, 5b sind mehrere, durch (nicht dargestellte) Federelemente lateral beabstandete Sensoren 12 angeordnet, wobei der laterale Abstand der Sensoren 12 sowohl bei kleinem Umfang der Tragstruktur (Fig. 3) als auch bei großem Umfang der Tragstruktur (Fig. 4) jeweils konstant ist.

Während die Magnetpole (N, S) der drehbaren Magnete 11, 11a bei der in Fig. 3 gezeigten Anordnung entgegengesetzt zu den Magnetpolen (N, S) der Permanentmagnete 10, 10a ausgerichtet sind, wodurch deren Magnetfeld maximal abgeschwächt wird, sind bei der in Fig.4 dargestellten Anordnung die Magnetpole (N, S) der drehbaren Magnete 11, 11a entsprechend den Magnetpolen (N, S) der Permanentmagnete 10, 10a ausgerichtet, wodurch deren Magnetfeld maximal verstärkt wird.

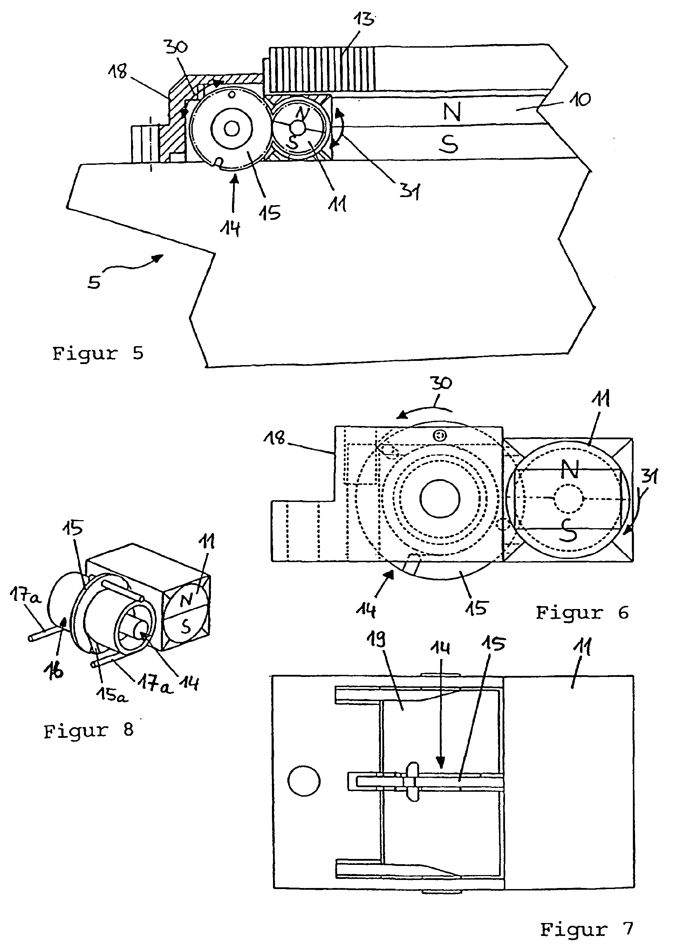

Ein Detailschnitt durch einen Parallelogrammträger 5 ist in Fig. 5 dargestellt. Der dem Permanentmagnet 10 zugeordnete Magnet 11 ist mittels eines Stellglieds 14 in Richtung Pfeil 31 drehbar, wobei das Stellglied 14 ein mit dem Magnet 11 kämmendes Zahnrad 15 aufweist. Das Stellglied 14 ist beispielsweise als mit dem Zahnrad 15 drehfest verbundene, in einer Aufnahme 18 gelagerte Welle ausgebildet. Oberhalb des drehbaren Magnets 11 und des Permanentmagnets 10 ist eine Bürste zum Schutz vor Verunreinigungen, Feuchtigkeit od. dgl. angeordnet.

Bei der in Fig. 6 in Seitenansicht und in Fig. 7 in Draufsicht dargestellten Ausführungsform eines elektrisch betriebenen Stellglieds 14 ist ein Elektromotor 19 zum Antrieb des mit dem Magnet 11 kämmenden Zahnrads 15 vorgesehen. Der Elektromotor 19 ist mit einem (nicht dargestellten), beliebigen elektrischen, elektronischen oder mechanischen Sensorelement zur Ermittlung des Umfangs der Tragstruktur 2 (Fig. 1) bzw. zur Ermittlung des lateralen Abstands der Parallelogrammträger 5 (Fig. 2) verbunden. Ein mechanisches Sensorelement kann beispielsweise in Form der Federwangen 5a, 5b (Fig. 3 und 4) verwirklicht sein.

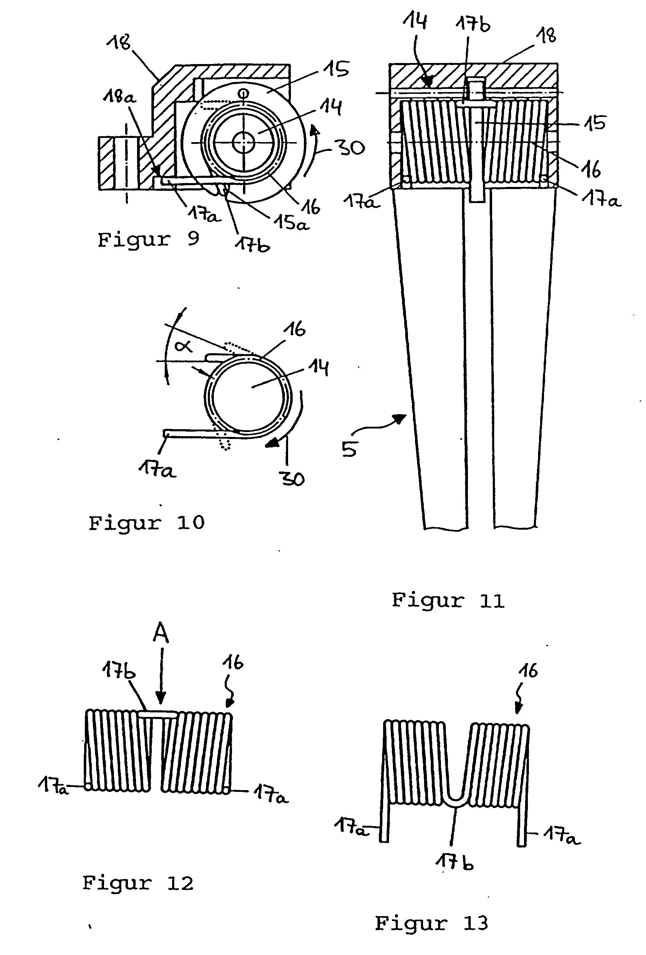

Das Stellglied 14 gemäß Fig. 8 weist ebenfalls ein mit dem Magnet 11 kämmendes Zahnrad 15 auf und ist rein mechanisch angetrieben, indem eine die (nicht dargestellten) Tragarme bzw. Parallelogrammträger lateral elastisch nach außen vorbelastende Schraubenfeder 16 die bei einem lateralen Annähern der Tragarme bzw. der Parallelogrammträger bzw. einem lateralen Entfernen derselben die mit ihrer Kompression bzw. Expansion verbundene Längenänderung in eine Drehbewegung des Stellglieds 14 bzw. des mit diesem drehfest verbundenen Zahnrads 15 umsetzt. Hierzu ist die Schraubenfeder 16 koaxial zum Stellglied 15 angeordnet und greift mit zwei Stützenden 17a in eine innenseitig der Aufnahme 18 ausgeformte Ausnehmung 18a ein (Fig. 9), während ein zentraler Wirkabschnitt 17b der Schraubenfeder 16 in eine im Stellglied 14 bzw. Zahnrad 15 ausgeformte radiale Nut 15a eingreift.

Wird die Schraubenfeder 16 z.B. komprimiert, so stützen sich ihre Stützenden in der Ausnehmung 18a der Aufnahme 18 ab und der Wirkabschnitt 17b beginnt das Zahnrad 15 in Richtung Pfeil 30 zu drehen. Wird die Schraubenfeder 16 expandiert, so dreht sie das Zahnrad 15 in die entgegengesetzte Richtung. Derart ist der Drehwinkel α (Fig. 10) des Stellglieds 14 proportional zur Längenänderung der Schraubenfeder 16. Der Drehwinkel α des Stellglieds 14 kann durch eine geeignete Übersetzung, z.B. durch Variation der Verzahnung des Zahnrads 15 bzw. durch Variation dessen Durchmessers, einen größeren, einen kleineren oder den gleichen Drehwinkel α auf den Magnet 11 übertragen.

In Fig. 11 ist die koaxial zum Stellglied 14 angeordnete Schraubenfeder 16 nochmals in Draufsicht dargestellt, wobei ihr Wirkabschnitt 17b mit dem Zahnrad 15 in Verbindung steht und ihre Stützenden 17a sich in der (geschnitten dargestellten) Aufnahme 18 abstützen. Bei einer solchen Ausführungsform mit rein mechanischem Antrieb des Stellglieds 14 können die Schraubenfedern 16 zusätzlich zu den die Parallelogrammträger 5 lateral nach außen vorbelastenden Federwangen 5a, 5b (Fig. 3 und 4) vorgesehen sein.

Fig. 12 und 13 sind Seitenansichten der Schraubenfeder 16 vor der Montage entnehmbar.

### Bezugszeichenliste

- 1: Zugteil
- 2: Tragstruktur
- 2a, 2b: Manschette
- 3: Längsmittelachse des Zugteils
- 4: Tragarm
- 5: Parallelogrammträger
- 5a, 5b: Federwangen
- 6: Schwenkachse des Parallelogrammträgers
- 7: Schwenkachse des Tragarms
- 8: Stützrolle
- 9: Zentralhülse
- 10, 10a: Permanentmagnet
- 11, 11a: Magnet mit veränderbarem Magnetfeld
- 12: Sensor
- 13: Bürste
- 14: Stellglied
- 15: Zahnrad
- 15a: Nut
- 16: Schraubfeder
- 17a: Stützende der Schraubenfeder
- 17b: Wirkabschnitt der Schraubenfeder
- 18: Aufnahme
- 18a: Ausnehmung
- 19: Elektromotor
- 20: Verbindungseinrichtung
- 21: Kupplungseinrichtung
- 22: Kupplungsstück
- 23: Zentralkörper
- 25: Feldlinien des Magnetfeldes

## Patentansprüche

1. Vorrichtung zur Prüfung von Rohrleitungen aus ferromagnetischen Materialen, wie Pipelines, auf Fehler, Risse, Korrosion oder dergleichen, mit wenigstens einem Zugteil (1), einer am Zugteil (1) angeordneten, jeweils um senkrecht zur Längsmittelsachse (3) des Zugteils (1) angeordnete Achsen (7) schwenkbare, im Wesentlichen strahlenartig angeordnete Tragarme (4) aufweisenden Tragstruktur (2) mit veränderbarem Umfang und mehreren, am Umfang der Tragstruktur (2) angeordneten Permanentmagneten (10) zur Erzeugung eines Magnetfeldes und Sensoren (12), **dadurch gekennzeichnet, dass** zumindest einigen Permanentmagneten (10) ein weiterer Magnet (11) zugeordnet ist, dessen Magnetfeld hinsichtlich Richtung und/oder Stärke veränderbar ist, um das von den Permanentmagneten (10) erzeugte Magnetfeld in Abhängigkeit vom Umfang der Tragstruktur (2) bzw. in Abhängigkeit vom lateralen Abstand der Permanentmagnete (10) zu verstärken oder abzuschwächen.

2. Vorrichtung nach Anspruch 1, **dadurch gekennzeichnet, daß** jedem Permanentmagnet (10) ein Magnet (11) mit veränderbarem Magnetfeld zugeordnet ist.

3. Vorrichtung nach Anspruch 1 oder 2, **dadurch gekennzeichnet, daß** der Magnet (11) mit veränderbarem Magnetfeld als Permanentmagnet ausgebildet und mittels eines Stellglieds (14) drehbar ist, um die Richtung seines Magnetfeldes zu verändern.

4. Vorrichtung nach Anspruch 3, **dadurch gekennzeichnet, daß** das Stellglied (14) wenigstens ein mit dem Magnet (11) kämmendes Zahnrad (15) aufweist.

5. Vorrichtung nach Anspruch 3 oder 4, **dadurch gekennzeichnet, daß** das Stellglied (14) elektrisch angetrieben ist.

6. Vorrichtung nach Anspruch 5, **dadurch gekennzeichnet, daß** das Stellglied (14) mittels eines Elektromotors angetrieben ist, der mit wenigstens einem Sensorelement zur Ermittlung des Umfangs der Tragstruktur (2) bzw. des lateralen Abstands der Permanentmagnete (10) in Verbindung steht.

7. Vorrichtung nach Anspruch 3 oder 4, **dadurch gekennzeichnet, daß** das Stellglied (14) mechanisch angetrieben ist.

8. Vorrichtung nach Anspruch 7, **dadurch gekennzeichnet, daß** das Stellglied (14) mittels wenigstens einer Schraubenfeder (16) angetrieben ist, die am Umfang der Tragstruktur (2) die Tragarme (4) lateral elastisch nach außen vorbelastet und bei einem lateralen Annähern der Tragarme (4) bzw. einem lateralen Entfernen der Tragarme (5) die mit ihrer Kompression bzw. Expansion verbundene Längenänderung in eine Drehbewegung des Stellglieds (14) umsetzt.

9. Vorrichtung nach Anspruch 1 oder 2, **dadurch gekennzeichnet, daß** der Magnet (11) mit veränderbarem Magnetfeld als Elektromagnet, wie eine Induktionsspule, ausgebildet und mit einem variablen Strom beaufschlagbar ist, um die Stärke seines Magnetfeldes zu verändern.

10. Vorrichtung nach einem der Ansprüche 1 bis 9, **dadurch gekennzeichnet, daß** jedem am Umfang der Tragstruktur (2) angeordneten Permanentmagnet (10) in Längsrichtung ein weiterer Permamentmagnet (10a) zur Erzeugung eines im wesentlichen parallel zur Längsmittelachse (3) des Zugteils (1) verlaufenden Magnetfeldes zugeordnet ist.

11. Vorrichtung nach Anspruch 10, **dadurch gekennzeichnet, daß** beiden Permantmagneten (10, 10a) jeweils ein weiterer Magnet (11, 11a) mit veränderbarem Magnetfeld zugeordnet ist.

12. Vorrichtung nach einem der Ansprüche 1 bis 11, **dadurch gekennzeichnet, daß** die Permanentmagnete (10, 10a) und die Magnete (11, 11a) mit veränderbarem Magnetfeld an Parallelogrammträgern (5) angeordnet sind, die jeweils an zwei hintereinander angeordneten Tragarmen (4) angelenkt sind.

13. Vorrichtung nach Anspruch 12, **dadurch gekennzeichnet, daß** die Parallelogrammträger (5) mittels Federwangen (5a, 5b) elastisch lateral nach außen vorbelastet sind.

14. Vorrichtung nach Anspruch 13, **dadurch gekennzeichnet, daß** die Federwangen (5a, 5b) zur Aufnahme mehrerer lateral beabstandeter Sensoren (12) zur Überprüfung des Zustandes der Wandung der Rohrleitung dienen.

15. Vorrichtung nach Anspruch 14, **dadurch gekennzeichnet, daß** die Sensoren (12) mittels Federelementen zwischen den Federwangen (5a, 5b) angeordnet sind, wobei der laterale Abstand der Sensoren (12) bei jedem Umfang der Tragstruktur (2) jeweils konstant ist.

16. Vorrichtung nach einem der Ansprüche 1 bis 15, **dadurch gekennzeichnet, daß** die Tragarme (4) mit einer Federkraft radial nach außen vorgespannt sind.

17. Vorrichtung nach Anspruch 16, **dadurch gekennzeichnet, daß** die Tragarme (4) mittels jeweils eines Tellerfederpakets vorgespannt sind.

18. Vorrichtung nach einem der Ansprüche 12 bis 17, **dadurch gekennzeichnet, daß** jeder Tragarm (4) an seinem äußeren Ende im Bereich der Schwenkachse (6) des Parallelogrammträgers (5) und/oder jeder Parallelogrammträger (5) wenigstens eine Stützrolle (8) zur Führung an der Innenwand einer Rohrleitung aufweist.

19. Vorrichtung nach einem der Ansprüche 12 bis 18, **dadurch gekennzeichnet, daß** die Tragarme (4) und/oder die Parallelogrammträger (5) synchronisiert sind.

20. Vorrichtung nach Anspruch 19, **dadurch gekennzeichnet, daß** jeweils zwei benachbarte Tragarme (4) und/oder zwei benachbarte Parallelogrammträger (5) unter Gewährleistung eines um zwischen 1° und 5°, insbesondere zwischen 2° und 3°, voneinander abweichenden Winkels der Tragarme (4) zur Längsmittelachse (3) des Zugteils (1) synchronisiert sind.

21. Vorrichtung nach einem der Ansprüche 1 bis 20, **dadurch gekennzeichnet, daß** die Tragarme (4) über eine am Zugteil (1) festlegbare Zentralhülse (9) an diesem befestigt sind.

## Claims

1. Device for inspecting conduits made from ferromagnetic materials, such as pipelines, for faults, cracks, corrosion or the like, comprising at least one pulling element (1), a supporting structure (2) of variable circumference, disposed on the pulling element (1) for pivoting about axes (7) disposed perpendicular to the longitudinal central axis (3) of the pulling element (1) and comprising substantially radially disposed supporting arms (4), and with several permanent magnets (10) disposed at the circumference of the supporting structure (2) for generating a magnetic field, and with sensors (12), **characterized in that** at least some permanent magnets (10) are associated with a further magnet (11) with variable magnetic field for strengthening and/or weakening the magnetic field generated by the permanent magnet (10) in dependence on the circumference of the supporting structure (2) or in dependence on the lateral separation between the permanent magnets (10).

2. Device according to claim 1, **characterized in that** each permanent magnet (10) is associated with a magnet (11) with variable magnetic field.

3. Device according to claim 1 or 2, **characterized in that** the magnet (11) with variable magnetic field is a permanent magnet which can be rotated by an actuator (14) for changing the direction of its magnetic field.

4. Device according to claim 3, **characterized in that** the actuator (14) comprises at least one toothed wheel (15) engaging the magnet (11).

5. Device according to claim 3 or 4, **characterized in that** the actuator (14) is electrically driven.

6. Device according to claim 5, **characterized in that** the actuator (14) is driven by an electric motor communicating with at least one sensor element for determining the circumference of the supporting structure (2) or the lateral separation between the permanent magnets (10).

7. Device according to claim 3 or 4, **characterized in that** the actuator (14) is driven mechanically.

8. Device according to claim 7, **characterized in that** the actuator (14) is driven by at least one helical spring (16) which biases the supporting arms (4) laterally elastically outwardly at the circumference of the supporting structure (2) and which, during lateral approach of the supporting arms (4) or lateral separation of the supporting arms (5), converts the longitudinal change occurring during its compression or expansion into rotational motion of the actuator (14).

9. Device according to claim 1 or 2, **characterized in that** the magnet (11) with variable magnetic field is an electric magnet, such as an induction coil, which can be driven with a variable current for changing the strength of its magnetic field.

10. Device according to any one of the claims 1 through 9, **characterized in that** each permanent magnet (10) disposed at the circumference of the supporting structure (2) is associated, in the longitudinal direction, with a further permanent magnet (10a) for generating a magnetic field extending substantially parallel to the longitudinal central axis (3) of the pulling element (1).

11. Device according to claim 10, **characterized in that** each of both permanent magnets (10, 10a) has an associated further magnet (11, 11a) with variable magnetic field.

12. Device according to any one of the claims 1 through 11, **characterized in that** the permanent magnets (10, 10a) and the magnets (11, 11a) with variable magnetic field are disposed on parallelogram supports (5) each of which is pivoted on two supporting arms (4), disposed one behind the other.

13. Device according to claim 12, **characterized in that** the parallelogram supports (5) are elastically laterally outwardly biased via spring jaws (5a, 5b).

14. Device according to claim 13, **characterized in that** the spring jaws (5a, 5b) accept several laterally separated sensors (12) for inspecting the state of the conduit walls.

15. Device according to claim 14, **characterized in that** the sensors (12) are disposed between the spring jaws (5a, 5b) via spring elements, wherein the mutual respective lateral separation between the sensors (12) is constant for each supporting structure (2) circumference.

16. Device according to any one of the claims 1 through 15, **characterized in that** the supporting arms (4) are radially outwardly biased by a spring force.

17. Device according to claim 16, **characterized in that** each of the supporting arms (4) is biased by a disc spring set.

18. Device according to any one of the claims 12 through 17, **characterized in that** each supporting arm (4), at its outer end proximate the pivot axis (6) of the parallelogram support (5) and/or each parallelogram support (5) comprises at least one supporting roller (8) for guidance on the inner wall of a conduit.

19. Device according to any one of the claims 12 through 18, **characterized in that** the supporting arms (4) and/or the parallelogram supports (5) are synchronized.

20. Device according to claim 19, **characterized in that** pairs of neighboring supporting arms (4) and/or pairs of neighboring parallelogram supports (5) are synchronized to ensure angles between the supporting arms (4) and the longitudinal central axis (3) of the pulling element (1) which differ by between 1° and 5°, in particular between 2° and 3°.

21. Device according to any one of the claims 1 through 20, **characterized in that** the supporting arms (4) are mounted to the pulling element (1) via a central sleeve (9) which can be fixed to the pulling element (1).

## Revendications

1. Dispositif pour la détection des défauts, fissures, phénoménes de corrosion ou autres dans des tuyauteries en matériau ferromagnétique, tels des pipe-lines, tel un écouvillon, comprenant au moins une partie tractrice (1), une structure porteuse (2) de circonférence variable placée sur la partie tractrice (1) présentant des bras (4) disposés essentiellement de façon radiale et pivotant chacun autour d'axes (7) perpendiculaires à l'axe médian longitudinal {3} de la partie tractrice (1) et plusieurs aimants permanents (10), disposés sur la circonférence de la structure porteuse (2) pour générer un champ magnétique et des capteurs (12), **caractérisé en ce que**, à au moins quelques uns des aimants permanents (10), est affecté un aimant supplémentaire (11) dont le champ magnétique est variable dans son orientation et/ou dans sa force afin de renforcer ou d'affaiblir le champ magnétique généré par les aimants permanents (10) en fonction de la circonférence de la structure porteuse (2) ou en fonction de l'écartement latéral des aimants permanents(10).

2. Dispositif selon la revendication 1, **caractérisé en ce qu'**à chaque aimant permanent {10) est affecté un aimant (11) à champ magnétique variable.

3. Dispositif selon la revendication 1 ou 2, **caractérisé en ce que** l'aimant (11) à champ magnétique variable est constitué par un aimant permanent et s'oriente sous l'effet d'un actionneur (14) afin de changer la direction de son champ magnétique.

4. Dispositif selon la revendication 3, **caractérisé en ce que** l'actionneur (14) présente au moins une roue dentée (15) s'engrenant dans l'aimant (11).

5. Dispositif selon la revendication 3 ou 4, **caractérisé en ce que** l'actionneur (14) est entraîné électriquement.

6. Dispositif selon la revendication 5, **caractérisé en ce que** l'actionneur (14) est entraîné au moyen d'un moteur électrique associé à au moins un élément capteur pour déterminer la circonférence de la structure porteuse (2) ou l'écartement latéral des aimants permanents (10).

7. Dispositif selon la revendication 3 ou 4, **caractérisé en ce que** l'actionneur (14) est entraîné mécaniquement.

8. Dispositif selon la revendication 7, **caractérisé en ce que** l'actionneur (14) est entraîné par au moins un ressort cylindrique (16) qui imprime une précontrainte latérale élastique vers l'extérieur sur les bras (4) à la circonférence de la structure porteuse (2) et qui convertit la variation de longueur liée à la compression ou à l'expansion dans le cas d'un rapprochement latéral des bras (4) ou d'un éloignement latéral des bras {5) en un mouvement de rotation de l'actionneur (14).

9. Dispositif selon la revendication 1 ou 2, **caractérisé en ce que** l'aimant (11) à champ magnétique variable est formé par un électroaimant, telle une bobine d'inductance et peut être alimenté par un courant variable afin de modifier la force de son champ magnétique.

10. Dispositif selon une des revendications 1 à 9, **caractérisé en ce qu'**à chacun des aimants permanents (10) disposés sur la circonférence de la structure porteuse (2) est affecté un aimant permanent (10a) dans le sens longitudinal pour générer un champ magnétique essentiellement parallèle à l'axe médian longitudinal (3) de la partie tractrice (1).

11. Dispositif selon la revendication 10, **caractérisé en ce qu'**à chacun des deux aimants permanents (10, 10a) est affecté un aimant supplémentaire {11, 11 a) à champ magnétique variable.

12. Dispositif selon une des revendications 1 à 11, **caractérisé en ce que** les aimants permanents (10, 10a) et les aimants (11, 11a) à champ magnétique variable sont disposés sur des parallélogrammes porteurs (5), lesquels s'articulent chacun sur deux bras (4) placés l'un derrière l'autre.

13. Dispositif selon la revendication 12, **caractérisé en ce que** les parallélogrammes porteurs (5) sont précontraints de manière élastique latéralement vers l'extérieur au moyen de flasques élastiques (5a, 5b).

14. Dispositif selon la revendication 13, **caractérisé en ce que** les flasques élastiques (5a, 5b) servent de support à plusieurs capteurs (12) espacés latéralement pour contrôler l'état de la paroi de la tuyauterie.

15. Dispositif selon la revendication 14, **caractérisé en ce que** les capteurs (12) sont disposés entre les flasques élastiques (5a, 5b) au moyen d'éléments à ressort, l'écartement latéral des capteurs (12) restant constant pour chaque circonférence de la structure porteuse.

16. Dispositif selon une des revendications 1 à 15, **caractérisé en ce que** les bras (4) sont précontraints radialement vers l'extérieur avec une force de ressort.

17. Dispositif selon la revendication 16, **caractérisé en ce que** les bras (4) sont précontraints chacun par un ressort à disques.

18. Dispositif selon une des revendications 12 à 17, **caractérisé en ce que** chaque bras (4) présente à son extrémité extérieure au niveau du pivot (6) du parallélogramme porteur (5) et/ou chaque parallélogramme porteur (5) présente au moins un rouleau d'appui (8) pour le guidage sur la paroi intérieure d'une tuyauterie.

19. Dispositif selon une des revendications 12 à 18, **caractérisé en ce que** les bras (4) et/ ou les parallélogrammes porteurs (5) sont synchronisés.

20. Dispositif selon la revendication 19, **caractérisé en ce que** les bras voisins (4) et/ou les parallélogrammes porteurs voisins (5) sont synchronisés deux par deux sous garantie d'un angle divergent compris entre 1° et 5° et plus particulièrement entre 2° et 3° formé par les bras (4) avec l'axe médian longitudinal (3) de la partie tractrice (1).

21. Dispositif selon une des revendications 1 à 20, **caractérisé en ce que** les bras (4) sont fixés à la partie tractrice (1) par l'intermédiaire d'une douille centrale (9) blocable sur ladite partie tractrice.
